# NEW EUROPEAN PATENT SPECIFICATION

(11) **EP 1 628 627 B2**
(45) Date of publication and mention of the opposition decision: **28.09.2011**
(45) Mention of the grant of the patent: 07.05.2008
(21) Application number: 04732985.9
(22) Date of filing: 14.05.2004
(51) Int. Cl.: A61Q 19/00, A61K 8/81, A61K 8/06

(54) **CLEAR OIL-IN-WATER EMULSIONS**
KLARE ÖL-IN-WASSER-EMULSIONEN
EMULSIONS TRANSLUCIDES HUILE-DANS-L'EAU

(30) Priority: 16.05.2003 EP 03076483
(43) Date of publication of application: 01.03.2006
(73) Proprietor: Johnson & Johnson GmbH, 40474 Düsseldorf (DE)
(72) Inventor: STORK, Anja, 50733 Köln (DE); ARIF, Ambareen, CH-8002 Zürich (CH); MUTTI, Bruna, 40545 Düsseldorf (DE)
(74) Representative: Metten, Karl-Heinz
(86) International application number: PCT/EP2004/005225
(87) International publication number: WO 2004/100862

(56) References cited:
- EP-A- 0 392 426
- US-A- 5 270 461
- US-A- 5 306 485
- US-A- 5 827 920
- NOVEON TDS-114: 'Introducing Pemulen Polymeric Emulsifiers' POLYMERS FOR PERSONAL CARE no. 9/99, 2001,
- TARA E. GOTTSCHALCK ET AL: 'INTERNATIONAL COSMETIC INGREDIENT DICTIONARY AND HANDBOOK', vol. 1, part 10 ED 2004, THE COSMETIC, TOILETRY, AND FRAGRANCE ASSOCIATION, WASHINGTON, D.C. page 32

## Description

### Field of the invention

This invention relates to clear oil-in-water emulsions with an electrosteric stabilizer.

### Background of the Invention

A clear and transparent appearance of personal care and cosmetic products has become an important product feature as the consumer associates it with attributes such as pureness, mildness, cleanliness, freshness, lightness and often possessing cooling properties. Clear products are used in a wide variety of personal care products for adults and babies. Another benefit of a clear appearance, in combination with a transparent packaging, is that the consumer is readily able to view and inspect the product. One type of personal care products are emulsions which find a wide variety of uses such as cleansers, caring products such as moisturizers, applicators of active ingredients such as sun-care products, anti-aging products, etc.

From the US 5,306,485 a suncare composition can be derived comprising a topical carrier and a sunscreen component which consists of octocrylene, titanium dioxide and additional sun screen actives. The topical carrier, for example, can be an oil-in-water emulsion. With these suncare compositions enhanced protection of the skin from the effects of ultraviolet radiation can be achieved. However, by nature a sunscreen or suncare composition comprises a variety of chromophores which do not allow to obtain a clear and transparent sunscreen composition. In addition, according to US 5,306,485 the choice of emulsifier is not critical so that a wide variety of non-ionic, cationic, anionic and zwitterionic emulsifiers can be employed.

In US 5,270,461 benzoic acid esters of an ethoxylated methyl glucoside and of a propoxylated methyl glucoside are disclosed which are suited as foam modifiers, emollients, conditioners and clarifiers. These two esters have an affinity for water/propylene glycole/glycerine mixtures while maintaining clarity. Both benzoic esters of US 5,270,461 shall have the ability to solubilize both hydrophilic and hydrophobic materials. However, no further applications of these or other esters are disclosed. US 5,270,461 explicitly only refers to a shampoo which comprises ammonium lauryl sulfate, sodium cocoyl isothionate, quaternium 75, cocoamide DEA, propylene glycole and water.

EP 0 392 426 B1 discloses a hand and body lotion in the form of a gelled dispersion which is free of potentially skin-irritating emulsifying agents which are normally present in lotion emulsions. This emulsifier-free hand and body lotion in the form of a clear gel dispersion comprises a gelled water system, a particulate carrier powder material of a cross-linked hydrophobic copolymer and at least one active ingredient. The active ingredient, that is the oil phase, is entrapped within the polymeric powder carrier and is therefore not in admixture directly with the water phase. The powder carrier material itself constitutes the interface between the water and the oil phase. The oil phase is only released when applied and rubbed into the skin furnishing a conventional creamy lotion emulsion. The particulate polymeric carrier of EP 0 392 426 B1 is a highly cross-linked polymethacrylate polymer. In order to even improve the clarity of the gelled water system an additional chelating agent is needed.

State of the art clear emulsions that are commercially available are water-in-oil type formulations, which contain specific emulsifiers. Emulsifiers are surface-active ingredients, which lower the surface tension of the formulation and which are needed to emulsify water and oil phases to an emulsion. The selection of emulsifiers that form clear emulsions is very limited. Commonly used emulsifiers for clear emulsions are silicone emulsifiers of the following type: polymers of dimethyl polysiloxane with PEG and/or PPG chains.

Classic emulsifiers are components belonging to the group of surfactants having hydrophilic and lipophilic portions and being capable of enclosing oil droplets with their lipophilic portions or, vice versa, water droplets with their hydrophilic portions. However, due to their surface activity, standard emulsifiers may interact with skin lipids thereby causing skin irritation. Therefore, standard emulsifiers are known to have a certain skin irritation potential, although some to a lesser extend than others. Additionally it is know that classic emulsifiers produce a tacky and sticky feeling on skin and hamper the interaction of lipophilic materials in the formulations with the skin. Therefore it is a desirable goal to reduce or even omit classic emulsifiers in cosmetic products.

So-called electrosteric stabilizers have been developed that build emulsions by a mechanism other than that of a standard emulsifier. These are water-soluble or water-swellable polymers with polar functionalities. Typical electrosteric stabilizers are polymeric carboxylic acids, in particular homo- or copolymers of acrylic acid or derivatives thereof. In this type of emulsions, the oil phase is entrapped in the polymeric network of the electrosteric stabilizer.

Quite unexpectedly, ithas been found that it is possible to build clear emulsions based on electrosteric stabilizers. Given the fact that only a very limited selection of silicone-based emulsifiers allow building clear emulsions, it is not obvious that this would also be possible with electrosteric stabilizers.

### Summary of the invention

The present invention concerns a clear oil-in-water emulsion according to the features of claim 1.

Other oily components may be selected from natural oils, fatty acid esters, ethers mono, di- and triglycerides, cyclic, branched or linear hydrocarbons, linear or branched fatty alcohols (Guerbet alcohols), and mixtures thereof.

The electrosteric stabilizer is a copolymer of poly-acrylate and C₁₀₋₃₀ alkylated polyacrylate.

The polyol in particular is a polyhydroxy alkane or -cycloalkane.

The emulsions according to the invention are substantially free of surfactants. Substantially free means no or an amount of surfactant that is insufficient to emulsify the oil phase in the emulsion.

### Detailed description of the invention

As used herein the term 'clear' means that the emulsion is transparent or essentially transparent when present in a typical consumer product, allowing ready viewing of objects behind it (when packed in a transparent container or packaging). In particular, the formulations of the present invention have a transparency (T % as measure unit) of T≥95%. The transparency may be measured with a UV/VIS double ray spectrometer at a wavelength of 800 nm.

### Oil Phase

The emulsions of the present invention contain silicone oils. The latter are excellent alternatives for traditional oils that are frequently used in personal care formulations. The use of formulations containing oils often leaves the user the impression of greasiness, tackiness, oiliness, or coating. Silicone oils on the other hand, having the benefits of traditional oils, lack these unfavorable properties and leave a tactile impression of softness, smoothness, and lightness. In recent years, silicone oils therefore have found use in many personal care formulations. Non-volatile as well as volatile silicone oils may be used, the latter being preferred over volatile silicone oils. Non-volatile silicones provide a lasting tactile impression, and tend to form a stable oil layer on the skin. If desired, volatile silicones may be used in combination with non-volatile silicones to impart desired esthetic properties, but preferably the emulsions should contain sufficient non-volatile silicone to provide a skin barrier layer.

Particular silicone oils are, for example, linear or cyclic silicones, in particular linear or cyclic dialkyl- or alkylarylpolysiloxanes. Appropriate linear silicone oils are those having a viscosity in the range of 50 to 1,000 cPs, having a viscosity in the range of 200 to 500 cPs, in particular at about 350 cPs. Linear or cyclic dialkyl- or alkylarylpolysiloxanes comprise for example dialkyl polysiloxanes (also referred to as polyalkylsiloxanes), alkylaryl polysiloxanes (or polyalkylarylsiloxanes) and polyether-siloxane-copolymers. Particularly suitable silicone oils comprise trimethylsiloxy terminated dimethylpolysiloxanes (also referred to as dimethyl polysiloxane, polydimethylsiloxane or dimethicone), methylphenylpolysiloxanes, copolymers of methylphenylsiloxane and dimethylsiloxane. Appropriate cyclic silicone oils are e.g. cyclic dialkyl polysiloxanes, and cyclic alkylaryl polysiloxanes. Of particular interest among the latter are the cyclic dimethyl polysiloxanes (also referred to as cyclic dimethylsiloxanes or cyclomethicone) and cyclic methylphenyl siloxanes. Also included are the alkoxylated and quaternized analogs of the above-referred linear or cyclic silicone oils as well as any mixture thereof, in particular mixtures of linear and cyclic dialkyl- or alkylarylpolysiloxanes.

Preferred silicone oils comprise cyclic dimethylsiloxanes, i.e. the cyclomethicones, e.g. tetracyclomethicone, pentacyclomethicone, and linear dimethylsilicones, i.e. the dimethicones, including any mixture of these.

The total amount of silicone oils in the emulsions according to the invention may vary but generally is in the range of 0.1% - 20%, preferably 0.2% - 15%, more preferably 0.5% - 10% (all % w/w, relative to the total weight of the emulsion).

The oil phase may additionally contain suitable oil components which are skin-compatible oil components or component mixtures that are non water-mixable and which may, for example, be natural oils, fatty acid esters, mono-, di- or triglycerides, or other oils, or mixtures thereof. Preferably, the oils are liquid at ambient temperature, in particular are liquid at 20°C or at 25 °C. They can contain certain amounts of solid lipid components (e.g. fats) as long as the complete oily mixture is liquid at ambient temperature or at the temperatures mentioned above.

Other oils, which can be incorporated comprise natural oils or fats, or natural oil derivatives, in particular of vegetable origin. Examples are almond oil, soybean oil, sunflower oil, safflower oil, corn oil, canola oil, borage oil, evening primrose oil, grapeseed oil, wheat germ oil, avocado oil, jojoba oil, kernel oil, sesame oil, walnut oil, linseed oil, palm oil, olive oil, macadamia oil, castor oil, rapeseed oil, peanut oil, coconut oil, and turnip seed oil.

The oil phase may also contain mono-, di or triglycerides. These can be derived from saturated or unsaturated, linear or branch chained, substituted or unsubstituted fatty acids orfatty acid mixtures. Particular mono-, di-ortriglycerides are mono-, di- or tri-C₁₂₋₂₄ fatty acid glycerides, specifically mono-, di-or tri-C₁₆₋₂₀ fatty acid glycerides, for example glyceryl monostearate, distearate or tristearate. Mixtures of mono-, di- and triglycerides can be derived from fractions of fatty acids. An example of the latter are C₁₂₋₁₈ fatty acid mono-, di- and triglycerides mixtures.

Further oil components comprise oily components isolated from natural oils, in particular from the natural oils mentioned above i.e. pure triglycerides or mixtures thereof, or the latter components having been prepared chemically. These so-called trigycerides (or triacyl glycerines) are esters of glycerines with fatty acids or fatty acid mixtures, for example so called technical mixtures obtained by hydrolysis from fractions of oils or fats, or by fractioning fatty acid mixtures after hydrolysis.

The fatty acids in said triglycerides may be saturated or unsaturated, straight or branch chained, substituted or unsubstituted. Preferred triglycerides are those glycerines esters derived from fatty acids, either saturated or unsaturated, having from 10 to 60, in particular from 12 to 36, more particularly from 12 to 24, preferably from 16 to 20 carbon atoms. Preferred such fatty acids are, for example, palmitic, palmic, oleic, lauric, myristic, stearic, hydroxystearic, behenic acid, or mixtures thereof. Within this group the triglycerides derived from saturated fatty acids are of particular interest.

They can also be mixed esters, i.e. tri-esters of glycerine with different fatty acids. Further such triglycerides are glycerine tristearate, also referred to as stearin, glycerine tribehenate, glycerine tripalmitate, glycerine trilaurate, glycerine trioleate, glycerine trimyristate.

Further oil components are mono- or diglycerides, optionally in a mixture with the fats and oils mentioned herein, in particular with triglycerides. The mono- or diglycerides are derived from saturated or unsaturated, linear or branch chained, substituted or unsubstituted fatty acids or fatty acid mixtures. Particular mono- or diglycerides are mono- or di-C₁₂₋₂₄ fatty acid glycerides, specifically mono- or di-C₁₆₋₂₀ fatty acid glycerides, for example glyceryl monostearate, glyceryl distearate. Mixtures of mono-, di- and, optionally, triglycerides can be derived from fractions of fatty acids. An example of such mixture is a mixture of C₁₂₋₁₈ mono-, di- and triglycerides.

The oil phase may also comprise alkyl esters of fatty acids, wherein the alkyl group in the latter has from 1 to 30 carbon atoms, preferably from 12 to 24 carbon atoms. The fatty acids in said alkyl esters in particular are C₁₂₋₃₀ fatty acids, more in particular C₁₂₋₂₀ fatty acids. The alkyl groups in said esters preferably are derived from fatty alcohols or of mixtures thereof, which, for example, are obtained by high-pressure hydrogenation of technical mixtures of the methyl esters derived from fats or oils.

Preferred are the alkyl esters of C₁₆₋₂₄ fatty acids, more preferably from C₁₆₋₁₈ fatty acids, and C₁₋₃₀ fatty alcohols, preferably C₈₋₂₄ fatty alcohols, more preferably C₁₂₋₂₀ fatty alcohols. Examples are the C₁₆-C₄₀-alkyl stearates, in particular the C₂₀-C₄₀-alkyl stearates.

Further oils are liquid esters from linear, saturated or unsaturated C₆-C₂₂-fatty acids with linear or branched, saturated or unsaturated C₆-C₂₂-fatty alcohols respectively esters from branched C₆-C₁₃-carboxylic acids with linear or branched, saturated or unsaturated C₆-C₂₂-fatty alcohols.

Examples of oil components of the ester type are the following: decyl oleate, coco caprylate/-caprate (available under the tradename Cetiol^{®} SN), hexyl laurate, myristyl isostearate, myristyl oleate, cetyl isostearate, cetyl oleate, stearyl isostearate, isostearyl myristate, isostearyl palpitate, isostearyl stearate, isostearyl isostearate, isostearyl oleate, isostearyl oleate, oleyl myristate, oleyl isostearate, oleyl oleate, oleyl erucate , behenyl isostearate, erucyl isostearate, erucyl oleate. Further oil components are the esters from linear C₆-C₂₂-fatty acids with branched alcohols, in particular 2-ethylhexanol (Cetiol^{®} 868), esters from branched C₆-C₂₂-fatty acids with linear alcohols, esters from C₁₈-C₃₈-alkylhydroxycarboxylic acids with linear or branched C₆-C₂₂-fatty alcohols, esters from linear and/or branched fatty acids with multifunctional alcohols (e.g. propylene glycol, dimerdiol or trimertriol) and/or Guerbet alcohols, as well esters from C₆-C₂₂-fatty alcohols and/or Guerbet alcohols with aromatic carboxylic acids, in particular benzoeic acids, esters from C₂-C₁₂-dicarboxylic acids with linear or branched alcohols having 1 to 22 carbon atoms (e.g. dioctyl malates).

Of particular interest in this regard are, e.g. stearyl stearate, palmityl stearate, stearyl behenate, cetyl stearate, cetyl behenate, cetyl palmitate, cetearyl behenate, behenyl behenate, stearyl heptanoate, stearyl octanoate, myristyl myristate, myristyl palmitate, myristyl stearate, myristyl isostearate, myristyl oleate, cetyl isostearate, cetyl oleate, stearyl isostearate, stearyl oleate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl isostearate, behenyl oleate, erucyl isostearate.

Of further interest are esters of C₁₈-C₃₈-alkylhydroxycarbonic acids with linear or branched C₆-C₂₂-fatty alcohols, esters of linear and/or branched fatty acids with polyalcohols (e.g. propylene glycol, dimerdiol or trimertriol) and/or Guerbet alcohols.

### Fatty alcohols

Other oil components that may be used comprise fatty alcohols. Fatty alcohols comprise, for example, C₁₂-C₅₀-fatty alcohols, in particular the C₁₂-C₂₄-fatty alcohols, more in particular the C₁₆-C₂₂-fatty alcohols that are derived from natural fats, oils or waxes such as, for example, myristyl alcohol, 1-pentadecanol, cetylalcohol, 1-heptadecanol, stearyl alcohol, 1-nonadecanol, arachidyl alcohol, 1-heneicosanol, behenyl alcohol, brassidyl alcohol, lignoceryl alcohol, ceryl alcohol, myricyl alcohol, lauryl alcohol, capryl alcohol, caprinyl alcohol, cetyl alcohol, palmoleyl alcohol, isostearyl alcohol, oleyl alcohol, elaidyl alcohol, petroselinyl alcohol, arachidyl alcohol, gadoleyl alcohol, erucyl alcohol, including mixtures thereof such as cetearyl alcohol, C_{12/13} fatty alcohol, as well as Guerbet alcohols, the latter being based on fatty alcohols having from 6 to 18, in particular from 8 to 10 carbon atoms. Preferred for use in the present invention, are saturated, straight or branch chained fatty alcohols. However also unsaturated, straight or branch chained alcohols can be used, optionally in a mixture with saturated alcohols.

Mixtures of fatty alcohols can evidently also be used, including fatty alcohol fractions obtained from the reduction of the corresponding fatty acid fractions, for example from the reduction of methyl ester fatty acid fractions. These may be derived from naturally occurring oils orfats such as, for example, almond oil, soybean oil, sunflower oil, safflower oil, corn oil, canola oil, borage oil, evening primrose oil, grapeseed oil, wheat germ oil, avocado oil, jojoba oil, sesame oil, walnut oil, linseed oil, palm oil, olive oil, castor oil, macadamia oil, rapeseed oil, peanut oil, coconut oil, and turnip seed oil. The fatty alcohols from which these products are derived can be saturated, straight or branch chained fatty alcohols. However also unsaturated, straight or branch chained alcohols can be used, optionally in a mixture with saturated alcohols. The fatty alcohols in particular are derived from natural fats, oils or waxes.

### Fatty acids

The oil phase may also contain C₁₄-C₄₀-fatty acids, including mixtures thereof. Of particular interest are the C₁₆-C₃₀-fatty acids. These comprise, for example, myristic-, pentadecanoic-, palmitic-, margaric-, stearic-, nonadecanoic-, arachic-, behenic-, lignoceric-, cerotic-, melissic-, erucaic-, elaeostearic-, oleic-, lonolenic-, lauric acid as well as substituted fatty acids, e.g. hydroxy-substituted fatty acids such as, for example, 12-hydroxystearic acid, and the amides or monoethanolamides of these fatty acids.

### Dialkyl(ene) ethers

The compositions may also contain dialkyl(ene) ethers which can be symmetric or asymmetric, straight or branch chained, saturated or unsaturated. Preferred are saturated C₆-C₃₀-dialkylethers, in particular C₆-C₂₄-dialkylethers. More preferred are C₆-C₂₀-dialkylethers, and particularly preferred are distearylethers and dibehenylethers. Dialkylethers of shorter chain length can also be used such as, for example, di-n-octylether, di-(2-ethylhexyl)-ether, laurylmethylether or octylbutylether, didodecylether.

These ethers can be obtained from the appropriate fatty alcohols in the presence of an acid catalyst following art-known procedures. Typical examples are ethers or mixed ethers derived from the alcohols mentioned herein above in the section 'fatty alcohols', including any mixtures thereof.

### Dialkyl(ene) carbonates

The dialkyl(ene) carbonates that can be used are symmetric or asymmetric, straight or branch chained, saturated or unsaturated. Preferred dialkyl(ene) carbonates are linear or branch chained, saturated or unsaturated C₁₄-C₃₀-dialkyl (ene) carbonates. More preferred are C₁₆-C₂₄-dialkyl carbonates and amongst these the saturated linear C₁₆-C₂₂-dialkyl carbonates. Particularly preferred is distearyl carbonate. Suitable dialkyl(ene) carbonates, are, for example, dihexyl-, dioctyl-, di-(2-ethylhexyl)- or dioleylcarbonate.

These dialkyl(ene) carbonates can be obtained by re-esterification of dimethyl- or diethyl carbonates with the corresponding hydroxy compounds following art-known procedures. Typical examples of dialkyl(ene) carbonates are re-esterification products of dimethyl- and/or diethyl carbonate with derived from the alcohols mentioned herein above in the section 'fatty alcohols', including any mixtures thereof.

### Dicarboxylic acids

Dicarboxylic acids that can be used are, for example, C₉-C₃₄-dicarbonic acids. These comprise, for example, octadecanedioic acid, tetratridecanedioic acid, etc. Of particular interest are the azelainic acids, which are C₉-dicarboxylic acids.

### Hydroxy fatty alcohols

The hydroxy fatty alcohols used in the compositions are saturated or unsaturated, straight chain or branched. Preferred are C₁₂-C₃₀-hydroxy fatty alcohols, at which the position of the hydroxy-substituent depends upon the synthesis route and the starting materials that have been used. Included are, for example, 1,10-decanediol, 1,2-hexadecanediol, 12-hydroxystearyl alcohol or hydroxy-Guerbet alcohols. Particularly preferred is 12-hydroxystearyl alcohol.

### Further oil components

Further oily components, which can be used comprise, mineral and paraffin oils and synthetic oils, either aliphatic or aromatic, as well as mixtures thereof.

Still further oil components that are hydrocarbons comprise, for example, squalane, squalene, paraffine oils, isohexadecane, isoeicosane or polydecene as well as dialkylcyclohexanes.

The total amount of the oily component in the emulsions of the invention, i.e. including all lipid components such as the silicone oils mentioned herein, may vary but generally is in the range of 2 - 70 %, preferably in the range of 5 - 50%, more preferably of 10 - 30% (w/w, relative to the total weight of the emulsion).

### The aqueous phase

The aqueous phase can be water but in most instances may contain further water soluble ingredients in particular any of the water-soluble agents mentioned hereinafter.

The emulsions of the present invention are for personal care applications, in particular for cleansing or treating skin, and therefore the pH of the emulsions is adjusted to be equal or near that of skin. In particular the pH will be adjusted to provide the emulsions with a pH of from about 4 to about 7, preferably from about 4.5 to about 6.5 more preferably about pH 5.5. The pH can be adjusted by adding suitable organic and inorganic acids or bases in amounts effective to provide such pH values Suitable buffer systems having a pH within the desired pH ranges can also be added. The pH-adjusting agent may be added to the aqueous phase prior to the emulsification process, or after the emulsification process or in both instances.

The emulsions of the present invention, wherein the electrosteric stabilizer is a polymeric carboxylic acid, may be more acidic than desired. Usually in that instance, a suitable base or buffer is added to bring the emulsions to the desired pH, in particular to a pH within the ranges as mentioned above. Suitable bases comprise organic amine bases, preferably amines that are skin-compatible, e.g. are relatively non-irritating, and which preferably have a relatively low equivalent weight (e.g. < 300), for example, triethanolamine, trimethylamine, triethylamine, tromethamine, aminomethyl propanol, tetrahydroxy ethylene diamine, including mixtures thereof. Inorganic bases, including alkali metal hydroxides such as NaOH, can also be used.

### Polyols or Hydroxy Acids

The emulsions of the invention furthermore contain one or more polyols or hydroxy acids or their salts. Suitable polyols for use in the emulsions of the invention comprise water-dissolvable polyols that form a clear aqueous solution. The polyols or hydroxy acids function to adjust the refractive index of the aqueous phase but may additionally serve other purposes in the emulsions, for example, they may function as a skin moistener, humectant, or emollient.

The polyol in particular is a polyhydroxy alkane or cycloalkane, or a water-soluble derivative thereof. Examples of the latter include, for example, water-soluble polyol esters or ethers, in particular of the groups of polyols or of the specific polyols mentioned herein. Of particular interest are those polyols with vicinal hydroxy functions, and preferred are the polyols of the glycol type, i.e. polyols having an ethylene glycol moiety, examples of such polyols being lower alkylene glycols such as ethylene, propylene, butylene, pentylene, and hexylene glycol, including oligomeric derivatives thereof such as diethylene glycol, triethylene glycol, or other water-soluble polyethylene glycols or water-soluble polypropylene glycols. Other examples of polyols for use in the emulsions of the invention are polyols such as glycerine, 1,2,4- butane triol,1,2,6-hexane triol, sorbitol, polyoxyethylene sorbitol, cyclohexanediol and the like. Also included are polyol mixtures. Of particular preference are ethylene glycol, propylene glycol and glycerine, including mixtures thereof.

Also water-soluble hydroxy acids and their salts can be used, e.g. glycolic, lactic and citric acid and the salts thereof, the latter in particular being derived from the suitable bases mentioned herein. Mixtures of polyols and hydroxy acids may also be used.

The polyols or hydroxy acids are added to adjust the refractive index of the aqueous phase to make it as close as possible to that of the oil phase. The refractive indices of both phases should be similar and in particular should not differ more than about 0.005, in particular not more than about 0.003 or about 0.002, preferably not more than about 0.0012.

The quantity of the polyol that is added therefore is selected such that the refractive index of the aqueous face meets these criteria.

In particular embodiments of the invention, the refractive index of the emulsion is in the range of from 1.3 to 1.5, in particular from 1.35 to 1.45, more in particular from 1.375 to 1.425, further in particular from 1.390 to 1.410.

The total amount of the polyols or hydroxy acids in the emulsions of the invention is in the range of 20 % - 60%, preferably in the range of 30 % - 50 %, more preferably of 35 % - 45 % (w/w, relative to the total weight of the emulsion).

### Electrosteric stabilizers

The clear oil-in-water emulsions of this invention contain so-called 'electrosteric stabilizers' that are polymeric components having polar groups that function to enclose oil droplets in a polymeric network. As such, the electrosteric stabilizers function as emulsifiers that provide an alternative for the classic emulsifiers of the surfactant type. Preferably, the electrosteric stabilizer is composed of a hydrophilic polymeric network that has hydrophobic cavities.

Of particular interest for use in the emulsions of the invention are those electrosteric stabilizers that are cross-linked carboxylic acid copolymers that contain monomers derived from acrylic acid, substituted acrylic acids, and salts and esters thereof, wherein the cross-linking agent contains two or more carbon-carbon double bonds. Preferably, the cross-linking agent is derived from a polyhydric alcohol.

Substituted acrylic acids may be substituted at the double bond carbon positions with one, two or three substituents independently selected from C₁₋₄alkyl,-cyano and carboxyl. Of particular interest are acrylic acid, methacrylic acid, ethyacrylic acid monomers, with acrylic acid being preferred.

Herein, carboxylic acid polymers are cross-linked copolymers composed of two or more monomers selected from acrylic acid and acrylic acid derivatives, the latter being as defined in the previous paragraph. Cross-linked carboxylic acid copolymers comprise monomers which are a long chain alcohol (i.e., C₁₀₋₃₀) acrylate ester monomer or derivative thereof. As used herein, the term derivatives of acrylic acid or of acrylate short chain or long chain esters is meant to comprise acrylic acid or acrylic ester derivatives having one, two or three substituents on one or both of the double bond carbon atoms. These substituents, for example, may be independently selected from C₁₋₄alkyl, CN and COOH.

A preferred acrylic acid monomer or derivative thereof in these copolymers is acrylic acid, methacrylic acid, or ethacrylic acid, the former two being particularly preferred. The long chain alcohol acrylate ester monomer in these copolymers is selected from C₁₀₋₃₀ alkyl acrylate esters.

Examples of commercially available carboxylic acid copolymers include copolymers of C₁₀₋₃₀ alkyl acrylates with one or more monomers of acrylic acid, methacrylic acid, or one of their short chain (i.e., C₁₋₄alcohol) esters, wherein the cross-linking agent is an allyl ether of sucrose or pentaerythritol. These copolymers are known as acrylates/C10-30 alkyl acrylate crosspolymers and are commercially available as the Carbopol® series of products, e.g. Carbopol® 1342 or Carbopol® 1382, the Pemulen® series of products, e.g. Pemulen® TR-1, and Pemulen® TR-2 (available from B.F. Goodrich Company).

These emulsifiers are usually described as hydrophobically modified hydrophilic polymers having good emulsifying capability and yielding emulsions of good stability. Their molecular weight is not exactly known but is estimated to range from about 700,000 to about 3 or 4 billion.

The Pemulen® emulsifiers are most preferred in the emulsions of the present invention. An additional advantage is that some of the Pemulen® emulsifiers enable relatively lower viscosities, which is attractive for cleansing efficiency and to provide a positive tactile impression.

The cross-linking agent in both types of carboxylic acid polymers preferably is a polyalkenyl polyether of a polyhydric alcohol containing more than one alkenyl ether group per molecule, wherein the polyhydric alcohol contains at least 3 carbon atoms and at least 3 hydroxyl groups. Preferred cross-linkers are allyl ethers of sucrose or allyl ethers of pentaerythritol.

It has been found that it is possible to make clear emulsions with this type of stabilizers. This is unexpected since making clear emulsion is only possible with specific emulsifiers (in particular silicone-based emulsifiers).
An additional advantage associated with the use of electrosteric stabilizers and in particular of the he polymeric carboxylic acid emulsifiers mentioned herein, is that they provide good emulsification properties, but also sufficiently rapidly de-emulsify upon application to the skin to form an oil film and in particular a silicone oil film on the skin that provides a protective barrier layer with soothing and caring properties.

The emulsifier is employed in an amount effective to emulsify the silicone oil and other non-water-soluble oils that may be present in the emulsion, typically an amount ranging from 0. 05% to 1.0%, preferably from 0.05% to 0.8%, w/w relative to the total weight of the emulsion. Particularly preferred emulsions of the present invention contain from about 0.1 % to 0.8%, more preferably from 0.2% to 0.6%, most preferably from 0.3 to 0.5% emulsifier, based on the weight of the emulsion.

### Further components

The aqueous or the oil phase in the emulsions of the invention may contain further components customarily used in personal care products, which components may be hydrophilic or lipophilic in nature. For example the emulsions may contain active ingredients, perfumes, emollients, anti-microbial agents, chelating agents, fragrance; skin soothing aids; skin moisteners, humectants, or and the like ingredients.

The aqueous phase in the emulsions of the invention may be present in varying amounts, but usually is present in amounts that are in the range of 50 - 98 %, preferably in the range of 60 - 95 %, more preferably of 70 - 90 % (w/w, relative to the total weight of the emulsion).

The emulsions of the invention are made by an appropriate emulsification process. An oily mixture is made by mixing the silicone oil and all other lipid components with the electrosteric stabilizer and the refractive index of this mixture is determined. An aqueous mixture is made by mixing the required quantity of water all aqueous components are mixed and a polyol or hydroxy acid is added until the refractive index of the aqueous mixture comes close to that of the oily mixture. The difference in diffractive indexes should be as specified herein. Subsequently the oily and aqueous phases are emulsified using standard emulsification techniques. For example, the oily phase may be added to the aqueous phase upon stirring. The pH of the aqueous phase may be adjusted by adding a suitable amount of pH adjuster. Since usually the electrosteric stabilizer is a polymeric acid, the pH adjuster is a suitable base such as sodium hydroxide, and is added to neutralize the polymeric acid and to bring the pH to a desired value, e.g. to a pH within the ranges mentioned herein. This can be done prior to the emulsification step or after that step, or at both stages. After emulsification, some small amounts of water may be added to further adjust the refractive index.

Another aspect of the invention is that the process for making the new clear emulsions has particular advantages compared to same for classical clear emulsions based on traditional emulsifiers in that it the step for making the emulsion clear can be done at the end of the process which has advantages in scaling up.

The clear emulsions of this invention are emulsifier free (increased mildness). They are from the oil-in-water type and therefore are lighter, fresher, non-greasy skin feel vs. water-in-oil types. The emulsions are very stable and may contain up to 70% oil phase. They deliver superior moisturizing and protecting properties compared to ordinary white oil-in-water emulsions and parity efficacy vs. standard W/O clear emulsions. This is surprising, as usually W/O type formulations show better protecting and moisturizing properties.

The emulsions of the invention can be used in several product types, e.g. in cleansers, caring products, moisturizers, emulsions for application of active ingredients, etc., both for applications on adults and babies.

### Examples

### Example 1

| INCI | Weight [%] |
|---|---|
| Dimethicone | 6,00 |
| Cyclopentasiloxane | 10,00 |
| Acrylates/C10-30 Alkyl Acrylate Crosspolymer, Aqua | 0,40 |
| Glycerin | 40,00 |
| Propylene Glycol | 4,00 |
| Aqua | 37,05 |
| Parfum | 0,10 |
| Trimethylglycine | 2,00 |
| Sodium Methylparaben | 0,20 |
| Citric Acid | 0,15 |
| Sodium Hydroxide | 0,10 |
| Total | 100,00 |

An oily mixture is made by mixing the silicone oil and all other lipid components with the electrosteric stabilizer and the refractive index of this mixture is determined. An aqueous mixture is made by mixing the required quantity of water all aqueous components are mixed and glycerine is added until the refractive index of the aqueous mixture comes close to that of the oily mixture. The difference in diffractive indexes is kept equal to or below 0.005. Subsequently the oily and aqueous phases are emulsified by adding the oily phase to the aqueous phase upon stirring and the whole is allowed to homogenize by stirring during 5 min. After the emulsification step, neutralization of the electrosteric stabilizer and adjustment of the pH of the mixture to pH 5.5 by adding the required amount of sodium hydroxide (in aqueous solution). Subsequently, some small amounts of water are added to further adjust the refractive index to 1.3998 - 1.3999.

### Example 2

In a similar manner, the following formulation is prepared.

| INCI | Weight (%) |
|---|---|
| Dimethicone | 6.0 |
| Cyclopentasiloxane | 10.0 |
| Acrylates/C10-30 Alkyl Acrylate Crosspolymer, Aqua (Pemulen™ TR-1) | 0.4 |
| Glycerin | 40.0 |
| Propylene Glycol | 4.0 |
| Aqua | 39.5 |
| Sodium Hydroxide | 0.1 |
| Total | 100.0 |

## Claims

1. Clear oil-in-water emulsion comprising:
(a) a water phase;
(b) an oil phase comprising a silicone oil, optionally in admixture with another oily component;
(c) an electrosteric stabilizer, which is a copolymer of polyacrylate and C₁₀₋₃₀ alkylated polyacrylate and
(d) from 20 wt.-% to 60 wt.-%, relative to the total weight of the emulsion, of a polyol and/or hydroxy acid,
wherein the emulsion is substantially free of surfactants.

2. An emulsion according to claim 1 wherein the silicone oil is dimethicone, cyclodimethicone or mixtures thereof.

3. An emulsion according to claim 1 wherein the other oily components are selected from natural oils, fatty acid esters, mono, di- and triglycerides, cyclic, branched or linear hydrocarbons, linear or branched fatty alcohols (Guerbet alcohols), and mixtures thereof.

4. An emulsion according to any of claims 1 to 3 wherein the polyol is a polyhydroxy alkane or -cycloalkane.

5. An emulsion according to claims 1 wherein the emulsion comprises:
(a) from 50 % to 98% of a water phase;
(b) from 2 % to 70 % of an oil phase comprising from 0.1 % to 20 %, w/w relative to the total weight of the emulsion, of a silicone oil, optionally in admixture with another oily component;
(c) from 0.05 % to 2 % of an electrosteric stabilizer; and
(d) from 20 % to 60 % of a polyol and/or hydroxy acid, and
wherein the silicone oil is dimethicone or cyclopentasiloxane, or a mixture thereof, the polyol and/or hydroxy acid is glycerin and the electrosteric stabilizer is acrylates/ C₁₀₋₃₀ alkyl acrylate crosspolymer.

6. An emulsion according to any of claims 1 to 5 wherein the difference of the refractive indexes of the oil phase and the water phase is less than 0.005.

7. An emulsion according to any of claims 1 to 6 wherein the pH of the emulsion is in the range of pH 4 to pH 7.

## Patentansprüche

1. Klare Öl-in-Wasser-Emulsion, umfassend:
(a) eine Wasserphase;
(b) eine Ölphase, umfassend ein Silikonöl, optional in Beimischung mit einer anderen öligen Komponente;
(c) einen elektrosterischen Stabilisator, der ein Copolymer aus Polyacrylat und C₁₀₋₃₀ alkyliertem Polyacrylat ist, und
(d) 20 Gewichtsprozent bis 60 Gewichtsprozent, im Verhältnis zum Gesamtgewicht der Emulsion, eines Polyols und/oder einer Hydroxysäure,
wobei die Emulsion im wesentlichen frei von Tensiden ist.

2. Emulsion nach Anspruch 1, **dadurch gekennzeichnet, dass** das Silikonöl Dimethicon, Cyclodimethicon oder Gemische derselben darstellt.

3. Emulsion nach Anspruch 1, **dadurch gekennzeichnet, dass** die anderen öligen Komponenten ausgewählt werden aus natürlichen Ölen, Fettsäureestern, Mono-, Di- und Triglyceriden, zyklischen, verzweigten oder linearen Kohlenwasserstoffen, linearen oder verzweigten Fettalkoholen (Guerbet-Alkohole), und Gemischen derselben.

4. Emulsion nach einem der Ansprüche 1 bis 3, **dadurch gekennzeichnet, dass** das Polyol ein Polyhydroxyalkan oder -cycloalkan ist.

5. Emulsion nach Anspruch 1, **dadurch gekennzeichnet, dass** die Emulsion umfasst:
a) 50 % bis 98 % einer Wasserphase;
b) 2 % bis 70 % einer Ölphase, umfassend 0,1 % bis 20 % w/w, im Verhältnis zum Gesamtgewicht der Emulsion, eines Silikonöls, optional in Beimischung mit einer anderen öligen Komponente;
c) 0,05 % bis 2 % eines elektrosterischen Stabilisators; und
d) 20 % bis 60 % eines Polyols und/oder Hydroxysäure, und
wobei das Silikonöl Dimethicon oder Cyclopentasiloxane, oder ein Gemisch derselben darstellt, wobei das Polyol und/oder Hydroxysäure Glycerin darstellt und wobei der elektrosterische Stabilisator ein Acrylat / C₁₀₋₃₀ Alkylacrylat-Kreuzpolymer darstellt.

6. Emulsion nach einem der Ansprüche 1 bis 5, **dadurch gekennzeichnet, dass** die Differenz der Brechungsindizes der Ölphase und der Wasserphase geringer als 0,005 ist.

7. Emulsion nach einem der Ansprüche 1 bis 6, **dadurch gekennzeichnet, dass** der pH der Emulsion im Bereich von pH4 bis pH7 liegt.

## Revendications

1. Emulsion transparente huile-dans-l'eau comprenant:
(a) une phase aqueuse;
(b) une phase huileuse comprenant une huile silicone, éventuellement en mélange avec un autre composant huileux;
(c) un stabilisateur électrostérique, qui est un copolymére de polyacrylate et de polyacrylate d'alkyle en C₁₀-C₃₀; et
(d) de 20% en poids à 60% en poids par rapport au poids total de l'émulsion, d'un polyol et/ou d'un hydroxyacide, dans laquelle l'émulsion est sensiblement exempte de tensioactifs.

2. Emulsion selon la revendication 1, dans laquelle l'huile silicone est la diméthicone, la cyclodiméthicone ou des mélanges de ceux-ci.

3. Emulsion selon la revendication 1, dans laquelle les autres composants huileux sont choisis parmi les huiles naturelles, les esters d'acide gras, les mono-, di- et triglycérides, les hydrocarbures cycliques, ramifiés ou linéaires, les alcools gras linéaires ou ramifiés (alcools de Guerbet), et des mélanges de ceux-ci.

4. Emulsion selon l'une quelconque des revendications 1 à 3, dans laquelle le polyol est un polyhydroxyalcane ou un polyhydroxycycloalcane.

5. Emulsion selon la revendication 1, dans laquelle l'émulsion comprend :
(a) de 50 % à 98 % d'une phase aqueuse ;
(b) de 2 % à 70 % d'une phase huileuse comprenant de 0,1 % à 20 %, poids/poids par rapport au poids total de l'émulsion, d'une huile silicone, optionnellement en mélange avec un autre composant huileux ;
(c) de 0,05 % à 2 % d'un stabilisateur électrostérique ; et
(d) de 20 % à 60 % d'un polyol et/ou d'un hydroxyacide, et dans laquelle l'huile silicone est la diméthicone ou le cyclopentasiloxane, ou un mélange de ceux-ci, le polyol et/ou l'hydroxyacide est de la glycérine, et le stabilisateur électrostérique est un polymère croisé d'acrylates/acrylate d'alkyle en C₁₀₋₃₀.

6. Emulsion selon l'une quelconque des revendications 1 à 5, dans laquelle la différence des indices de réfraction de la phase huileuse et de la phase aqueuse est inférieure à 0,005.

7. Emulsion selon l'une quelconque des revendications 1 à 6, dans laquelle le pH de l'émulsion est dans la plage de pH 4 à pH 7.
